# EUROPEAN PATENT APPLICATION

(11) **EP 1 857 144 A2**
(43) Date of publication of application: **21.11.2007**
(21) Application number: 07007665.8
(22) Date of filing: 14.04.2007
(51) Int. Cl.: A61N 1/372, A61N 1/39

(54) **Semi-automatic atrial defibrillation system, implantable atrial defibrillator and portable communication device**

(30) Priority: 15.05.2006 US 383235
(71) Applicant: BIOTRONIK CRM Patent AG, 6341 Baar (CH)
(72) Inventor: Lian, Jie, Beaverton OR 97007 (US); Müssig, Dirk, West Linn OR 97068 (US)
(74) Representative: Lindner-Vogt, Karin L.

(57) **Abstract**

A semi-automatic atrial defibrillation system monitors activity of a heart, detects atrial fibrillation (AF), and cardioverts detected AF. The system includes an implantable atrial defibrillator capable of communicating with an external portable device that can further communicate with a remote service centre. The implantable atrial defibrillator is capable of detecting AF, automatically sending warning signal and diagnostic data to an external portable device after detection of AF, receiving commands from the external portable device, and cardioverting AF. The external portable device is capable of sending commands to the implantable atrial defibrillator, receiving data from the implantable atrial defibrillator, providing patient discernable warning signal and brief diagnostic information after detection of AF, and transmitting diagnostic information to the remote service centre. The external portable device is operable by the patient, who may self-control the delivery of atrial cardioversion therapy after receiving the AF warning message, or may contact the remote service centre for physician's advice before taking any action.

## Description

The present invention generally relates to a defibrillation system including an implantable atrial defibrillator for applying cardioverting electrical energy to the atria of a human heart in need of cardioversion. The present invention is more particularly directed to such a system having an external portable device capable of communicating with the implantable atrial defibrillator and further communicating with the remote service centre.

Atrial fibrillation (AF) is probably the most common cardiac arrhythmia. Although it is not usually a life threatening arrhythmia, it is associated with strokes thought to be caused by blood clots forming in areas of stagnant blood flow as a result of prolonged AF. In addition, patients afflicted with AF generally experience palpitations of the heart and may even experience dizziness or even loss of consciousness.

As used herein, AF will be used to ream atrial fibrillation, flutter, or tachycardia correctable by defibrillation, low energy cardioversion, antitachycardia pacing (ATP), or drugs.

AF occurs suddenly, and many times can only be corrected by a discharge of electrical energy to the heart. Implantable atrial defibrillators have become a reality to provide relief to patients suffering from occurrences of AF.

Prior art documents U.S. Pat. 5,207,219, U.S. Pat. 5,282,837 and U.S. Pat. 5,350,404 disclose implantable atrial defibrillators wherein AF is automatically detected and, when needed, cardioverting electrical energy is applied to the atria to terminate the AF episode and return the heart to normal sinus rhythm. An improvement was disclosed in U.S. Pat. 5,332,400, in which the atrial defibrillator provides a warning by delivery of electrical energy to the internal tissue of the patient. The energy being delivered is sufficient enough to be discerned by the patient, but low enough to avoid causing pain or other undesirable effects.

One common problem in the systems described above is that the cardioverting energy is automatically delivered to the patient (with or without warning) if AF is detected. Since AF is fairly well tolerated by most patients, a fully automatic atrial defibrillation modality may not be necessary for a great number of patients. It is possible that a large number of shocks would be delivered to treat short asymptotic self-terminating episodes of AF. In addition, false automatic AF detections may occur when the patient is not in AF. Furthermore, a patient may not be in a position to receive cardioversion therapy at a time when the committed therapy is to be delivered (for example, during driving). At other times, a patient may simply want to confirm the presence or absence of AF, or be able to determine that the AF detector of the patient's defibrillator agrees with patient's perception of the arrhythmia. Hence, there is a need for an atrial defibrillation system that allows the patient to control where and when to initiate the cardioversion therapy.

Two proposed atrial defibrillators, which require human interaction for cardioverting or defibrillating the heart, are disclosed in U.S. Pat. No. 3,952,750. Both of these proposed defibrillators require the patient to recognize the symptoms of AF with one defibrillator requiring a visit to a physician to activate the defibrillator and the other defibrillator requiring the patient to activate the defibrillator with an external magnet. Neither defibrillator includes an AF detector to detect atrial activity of the heart. As a result, these manually operated defibrillators provide no AF detection support for the patient. Furthermore, the external magnet is inconvenient to use and provides no feedback to inform the patient that the implantable atrial defibrillator is acting upon the external command.

An improvement is disclosed in U.S. Pat. 5,490,862, in which the atrial defibrillator provides a manual or patient activated modality that includes automatic AF detection to confirm the patient's own diagnosis. Such a confirmation ensures the cardioversion therapy being activated only if the AF is actually present, thus the patient will not be subjected to unnecessary cardioversion attempts which otherwise may cause discomfort to the patient and early depletion of the defibrillator power source. In U.S. Pat. No. 5,674,249, another atrial defibrillation system including a portable external communication device is disclosed. Such a portable communication device can transmit a command signal to the implantable defibrillator. The implantable defibrillator will transmit an acknowledgment signal back to the external device upon receipt of the command signal, and activates the cardioversion therapy only if the AF detector detects AF. The external device receives the acknowledgment signal and provides a perceptible indication responsive to receipt of the acknowledgment signal to the patient.

Several further improvements are recently disclosed. U.S. Pat. No. 6,006,132 discloses an implantable device that can generate messages indicative of operating stage operation status and transmit to the external device. The external device decodes the received messages and converts them to selected voice signals to audible sound. U.S. Pat. No. 5,999,851 discloses an atrial defibrillation system that enables a patient to select and activate AF detection without committing to cardioverting therapy. Alternatively, the atrial defibrillator may automatically determine if the atria are in fibrillation at predetermined times and initiate an antitachycardia therapy upon detection of AF without prior warning of the patient. U.S. Pat. 6,068,651 discloses an atrial defibrillator that includes a patient activated mode, a programmable safety timer, and means for deactivating the patient activated mode. The programmable safety timer starts counting down when AF is detected. If the safety timer times out before the patient activates delivery of an atrial defibrillation shock, the device will deactivate the atrial defibrillation function, and will not allow an atrial defibrillation shock to be delivered until the physician reactivates it with a programmer.

However, for all atrial defibrillation systems described above, there is one common drawback that is they all rely on the patient to recognize the symptoms of AF. In other words, the patient is responsible for AF detection whereas the implant device operates in passive mode that it cannot actively detect the AF or notify the patient. Although early atrial defibrillation is encouraged, in most cases, hours or even days elapse before the patient perceives the symptom. One of the reasons is that many AF episodes are asymptotic. Another reason is that many AF episodes initiate during the night when the patient is asleep.

Epidemiological studies of the natural history of AF have shown that paroxysmal AF frequently progresses to persistent AF even in the absence of underlying structural heart disease. The likelihood of successful restoration and maintenance of sinus rhythm following cardioversion, is inversely related to the preceding duration of the arrhythmia. These findings suggest that AF is a self-perpetuating arrhythmia. This is confirmed by electrophysiological studies that demonstrated that episodes of AF resulted in decreased atrial refractoriness favoring initiation and maintenance of the arrhythmia, i.e. AF begets AF. It has been reported that as little as 5 minutes of rapid atrial pacing was sufficient to significantly shorten the atrial effective refractory period. Thus, even if atrial defibrillation were performed as soon as patients were aware of the arrhythmia, atrial remodeling is unlikely to have been prevented.

Therefore, there is a need in the art for an atrial defibrillator that actively monitors the atrial rhythm, ensures early detection of AF and automatic warning of the patient, and meanwhile allows the patient has flexible control of the therapy.

According to the invention this object is achieved by (1) an implantable atrial defibrillator, (2) an external portable device, and (3) optionally a remote service centre, forming a system that, on one hand, can automatically provide warning and brief diagnostic information to the patient soon after the detection of the AF, while on the other hand, allows the patient manually control the AF therapy. Upon detection of the AF, the implant device automatically sends a warning signal to the patient. The atrial defibrillation therapy can be automatically activated by the implant device or manually activated by the patient. In both activation modes, patient can intervene the therapy through manual control of the external portable device. For an atrial defibrillator that operates in patient-activation mode, such a feature will provide the patient the opportunity to initiate cardioversion therapy soon after the onset of the AF to prevent atrial remodeling. For an atrial defibrillator that operates in automatic-activation mode, such a feature will enable the patient to be well prepared for the predictable cardioversion therapy, or the patient may have the option to manually turn off or delay the scheduled automatic atrial cardioversion when necessary. It is also desirable for an atrial defibrillation system to be able to transmit related diagnostic information to the patient's physician after automatic detection of the AF, so that the patient can get expert's advice before taking any action. This feature will provide the patient additional sense of security and confidence, particularly if the patient is asymptotic when the AF is detected.

As a particularly novel feature, the system provides for a semi-automatic mode, wherein the implantable atrial defibrillator is adapted to automatically detect an atrial fibrillation leading to a patient discernible warning signal enabling the patient to manually initiate AF therapy like atrial cardioversion or atrial defibrillation. Semi automatic means automatic detection (and warning) and manual control of therapy in an atrial defibrillation system (for both automatic and patient activation modes).

The present invention provides an atrial defibrillation system including an implantable atrial defibrillator including therapy intervention means for detecting and cardioverting AF of the patient. The means for detecting and cardioverting AF comprise an automatic atrial fibrillation detection unit and an atrial cardioversion/defibrillation unit. The therapy intervention means preferably further comprise an atrial stimulation unit. While the atrial stimulation unit is adapted to generate atrial stimulation pulses having a strength just enough to cause excitation of the atrial myocardium if the atrium is not refractory, the atrial cardioversion/defibrillation unit is adapted to generate cardioversion pulses and defibrillation shocks which are stronger than atrial stimulation pulses. The implantable atrial defibrillator comprises bidirectional data transceiver for bidirectional data communication between the implantable atrial defibrillator and the external device. The implantable atrial defibrillator may further comprise a wireless interface for communication with an external programmer.

The system also provides an external portable device dimensioned to be hand-held and including a communication unit to communicate with the implantable atrial defibrillator, and including another communication unit to communicate with the remote service centre. The communication unit serves for communication with the implantable atrial defibrillator and comprises a first external bidirectional data transceiver adapted to bi-directionally communicate with the implant's bidirectional data transceiver of the implantable atrial defibrillator. The external portable device comprises a human interface including an output unit for putting out signals like warning signals and an input unit for receiving commands entered by a patient. The human interface is connected to communication unit.

The system provides warning means for warning the patient that AF is present and there is a need for cardioversion. The warning means comprise the output unit of the external portable device. The output unit is adapted to generate a warning signal that may be presented to the patient in the form of visible messages, or audible messages, or discernible vibrations, through the external portable device.

The external portable device communicates with the implantable atrial defibrillator, which preferably generates a warning trigger signal after a programmable delay after the automatic detection of AF, preferably also after a programmed number of anti tachycardia pacing (ATP) attempts fail to terminate the AF. The warning trigger signal is transmitted to the external portable device and causes the external portable device to put out the patient discernible warning signal or a more comprehensive warning message. For the purpose of this disclosure warning signal is used as a more general term which also includes warning messages.

By communicating with the implantable atrial defibrillator, the external portable device may also display brief diagnostic information, such as the duration of the AF since its onset, the mean cycle length of the AF, and so on. If the implantable atrial defibrillator is programmed to automatic-activation mode, the external portable device may also provide the patient warning messages that an automatic atrial cardioversion is scheduled at specific time or after certain period of time. The warning message will be cleared once AF is either self-terminated or terminated by atrial cardioversion therapy.

The atrial defibrillation system according to the invention further provides patient control means for control of the delivery of the atrial cardioversion therapy. The patient control means comprise the input unit of the external portable device.

The input unit preferably is configurable in order selectively activate and deactivate command entering means like buttons. The external portable device is adapted to activate or deactivate entering means depending on signals received via the first external bidirectional data transceiver and thus to configure the human interface depending upon the patient's health state.

If the patient-activation mode is selected and the patient receives the warning of the presence of AF, the patient can operate the external portable device to send a command to the implantable atrial defibrillator to manually initiate the atrial cardioversion therapy. Upon receiving the command signal from the external portable device, the implantable atrial defibrillator will further check the presence or absence of AF, and the cardioverting energy will be delivered only if AF is confirmed by the implantable atrial defibrillator prior to the atrial cardioversion. If the automatic-activation mode is selected and the patient receives the warning of the presence of AF, the patient may optionally turn off or delay the scheduled automatic atrial cardioversion by operating the external portable device, which sends corresponding commands to the implantable atrial defibrillator.

The invention further provides remote monitoring means for remote monitoring the electrical activity of the heart and the status of the implantable atrial defibrillator. After receiving the warning of the presence of AF, the external portable device may operate in automatic mode or patient activated mode to send a command to the implantable atrial defibrillator to retrieve related diagnostic information, such as the device-recorded atrial intracardiac electrogram (IEGM). Then through a communication network, the external portable device can send the retrieved diagnostic information to the remote service centre for expert review. The patient thus can have the option to contact the service centre and obtain physician's recommendation before taking any action.

The atrial defibrillation system according to the invention comprises:
- an implantable atrial defibrillator including at least means for sensing electrical activity and delivering pacing pulses to atria of a heart, detecting atrial fibrillation, and cardioverting the atria of the heart in need of cardioversion; and
- an external portable device dimensioned to be hand-held and operable by the patient, including means to communicate with the implantable atrial defibrillator and means to communicate with the remote service centre; and
- an optional remote service centre including means to communicate with the external portable device.

The implantable atrial defibrillator for use in said system may further include implant's communication means including a telemetry unit for communicating with the external programmer and the external portable device, wherein implant's communicating means are adapted to
- receive commands and parameters from an external programmer; and
- transmit data to the external programmer; and
- receive commands from the external portable device; and
- transmit data to the external portable device.

The first communication means of the external portable device to communicate with the implantable atrial defibrillator include first external bidirectional data transceiver and are adapted to transmit commands to the implantable atrial defibrillator; and to receive data from the implantable atrial defibrillator.

The communication means of the external portable device to communicate with the remote service centre are adapted to transmit data to the remote service centre; and to receive data from the remote service centre.

The means for cardioverting the atria of the heart are adapted to apply cardioverting electrical energy to the atria of the heart that is in atrial fibrillation and include:
- means for checking the presence of atrial fibrillation prior to atrial cardioversion and abandoning the atrial cardioversion if atrial fibrillation is not confirmed; and
- means for delivering the cardioverting electrical energy in synchronous with a shockable R wave based on analysis of the ventricular sensed signals; and
- means for providing shock therapy to the ventricle in case a ventricular tachyarrhythmia being detected.

The data being transmitted from the external device and received by the implantable atrial defibrillator include at least one of but preferably all of the following commands further referred to as patient commands:
- a therapy trigger command that initiates atrial cardioversion therapy; and
- a therapy enable/disable command that disables or enable scheduled automatic atrial cardioversion therapy; and
- a therapy delay (or cancellation) command that delays (or cancels) scheduled automatic atrial cardioversion therapy; and
- a data request command that requests implantable atrial defibrillator send diagnostic data to the external portable device.

The data being transmitted from the implantable atrial defibrillator and received by the external portable device include at least one of but preferably all of:
- an acknowledgement signal indicating that commands sent by the external portable device are received by the implantable atrial defibrillator; and
- data indicating the operation status of the implantable atrial defibrillator; and
- a warning trigger signal indicating atrial fibrillation is detected by the implantable atrial defibrillator, said warning trigger signal being transmitted after a programmable time delay and after a programmable number of atrial antitachycardia pacing attempts, and
- an end of AF signal (eoAF signal) indicating termination of atrial fibrillation is detected by the implantable atrial defibrillator, and
- data indicating duration of the atrial fibrillation since its onset, mean cycle length of the atrial fibrillation, time interval to the scheduled automatic atrial cardioversion, snapshot of the recorded intracardiac atrial electrogram, and other related diagnostic information recorded by the implantable atrial defibrillator.

The data being transmitted from the external portable device and received by the remote service centre may include data received by the external portable device from the implantable atrial defibrillator, including at least one of:
- a duration of the atrial fibrillation since its onset,
- a mean cycle length of the atrial fibrillation,
- a time interval to the scheduled automatic atrial cardioversion,
- a snapshot of the recorded intracardiac atrial electrogram, and
- other related diagnostic information recorded by the implantable atrial defibrillator.

The data being transmitted from the remote service centre and received by the external portable device include an acknowledgement signal indicating that data sent by the external portable device are received by the remote service centre.

The external portable device may further include an input unit comprising a keypad or press switches or a touch screen to receive patient inputted commands. These patient-inputted commands include at least one of:
- a configuration command that configures the external portable device, including turning on or off certain outputs, and selecting specific communication protocols; and
- a service centre communication establishing command that establishes bi-directional communication between the external portable device and the remote service centre; and
- an implant communication establishing command that establishes bi-directional communication between the external portable device and the implantable atrial defibrillator.

The external portable device may further include an output unit that provides perceptible indication responsive to receipt of the data from the implantable atrial defibrillator and remote service centre, wherein the perceptible indication includes at least one of:
- a light-up or blinking of one or more light emitting diodes; and
- a text message displayed in a liquid crystal display; and
- an audible message played by a speaker; and
- a mechanical vibration generated by a vibrator.

The remote service centre includes means for receiving the data transmitted from the external portable device, storing the data into a database, sending acknowledgement back to the external portable device, and presenting the data to the patient's physician or responsible expert.

The various features and advantages of the present invention may be more readily understood with reference to the following detailed description taken in conjunction with the accompanying drawings in which:
FIG. 1 illustrates the block diagram of an implantable atrial defibrillator embodying the present invention, and its interfaces with an external programming device and an external portable device, which further communicates with the remote service centre.
FIG. 2 illustrates the block diagram of the external portable device embodying the present invention, and its interfaces with the implantable atrial defibrillator and the remote service centre.
FIG. 3 illustrates by way of a block diagram the operation of the atrial defibrillation system according to the invention in an automatic activation mode.
FIG. 4 illustrates by way of a block diagram the operation of the atrial defibrillation system according to the invention in a patient activation mode.

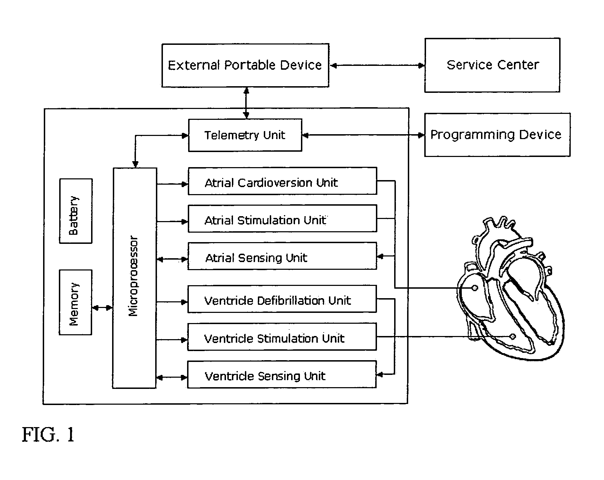

Referring now to FIG. 1, it illustrates an atrial defibrillation system embodying the present invention including an implantable atrial defibrillator shown in association with a schematically illustrated human heart in need of atrial fibrillation monitoring and potential cardioversion, an external programming device, and an external portable device, which further communicates with the remote service centre. For illustration purposes, the schematic block diagram of the implantable atrial defibrillator is described to include the dual-chamber pacemaker functionality.

In this device, a microprocessor and associated circuitry make up the controller, enabling it to output pacing pulses or cardioversion/defibrillation energy to the atrium and ventricle of the heart in response to sensed events and lapsed time intervals. The microprocessor communicates with a memory via a bi-directional data bus. The memory typically comprises a ROM or RAM for program storage and a RAM for data storage.

The device has atrial channels comprising atrial electrode, atrial lead, atrial sensing unit, atrial stimulation unit, and atrial cardioversion unit. The ventricular channels similarly comprise ventricular electrode, ventricular lead, ventricular sensing unit, ventricular stimulation unit, and a ventricular defibrillation unit. For each channel, the same lead and electrode are used for both sensing and pacing, while cardioversion/defibrillation may share the same electrode or use a different shock electrode. The sensing channels are used to control pacing and for measuring heart rate in order to detect cardiac arrhythmias. The device detects the occurrence and termination of an AF, for example, by measuring the atrial rate as well as possibly performing other processing on the data received from the atrial sensing unit.

The atrial cardioversion unit is interfaced to the microprocessor for delivering cardioverting energy to the atrium in need of cardioversion, in synchronous with a shockable R wave. As well known in the art, the microprocessor determines if an R wave is shockable by analyzing the ventricular rhythm based on the data received from the ventricular sensing unit to minimize the probability of ventricular arrhythmias induced by atrial cardioversion. Preferably, the atrial sensing unit further checks the activity of the atrium to confirm the presence of AF before discharging the cardioverting energy. A ventricular defibrillation unit is also provided to deliver shock therapy to the ventricle in case a ventricular tachyarrhythmia being detected by the ventricular sensing unit.

The external programming device is arranged to communicate with a telemetry unit, which is coupled to the microprocessor over a bi-directional bus. The telemetry unit may be of the type well known in the art for conveying various information which it obtains from the microprocessor to the external programming device, or for receiving programming parameters from the external programming device and then conveys to the microprocessor for storage in the memory.

In addition, the external portable device to be described hereinafter, can also communicate bi-directionally with the microprocessor through the telemetry unit comprising a first external bidirectional data transceiver. Preferably, the parameters that may be received from or sent to the external portable device are vastly limited as compared to the parameters which may be received from or sent to the external programming device.

To that end, the parameters which may be provided by the external portable device are preferably simple commands, such as initiate atrial cardioversion therapy, disable or enable automatic atrial cardioversion therapy, delay scheduled atrial cardioversion therapy by certain period of time, retrieve IEGM and other diagnostic information from the implantable atrial defibrillator, and so on. These commands set the implantable atrial defibrillator into one of a number of modalities wherein each modality is determined and controlled by parameters that can only be selected by a physician operating the external programming device.

The parameters which may be transmitted to the external portable device include simple acknowledgment to confirm receiving the commands from the external portable device, the signals warning the detection of AF or indicating the termination of AF, and some critical diagnostic information, such as the duration of the AF since its onset, the mean cycle length of the AF, the time interval to the scheduled automatic atrial cardioversion, the snapshot of the recorded IEGM, and so on.

In a preferred embodiment, the warning trigger signal is transmitted to the external portable device after a programmable delay after the automatic detection of AF. This will prevent generating warning messages to the patient in case of short and self-terminating AF episodes. Preferably, the implantable atrial defibrillator can also be programmed to transmit the warning signal only after programmed number of anti-tachycardia pacing (ATP) attempts failed to terminate the AF, indicating atrial cardioversion will be the preferred choice to terminate AF. After the warning trigger signal is transmitted to the external portable device, if AF is terminated by the automatic or manual atrial cardioversion, or self-terminates before the atrial cardioversion, the implantable atrial defibrillator also sends an eoAF signal to the external portable device to clear the warning signal being generated.

Furthermore, the device includes a depletable power source, such as a lithium battery, which provides power to the electrical components of the device.

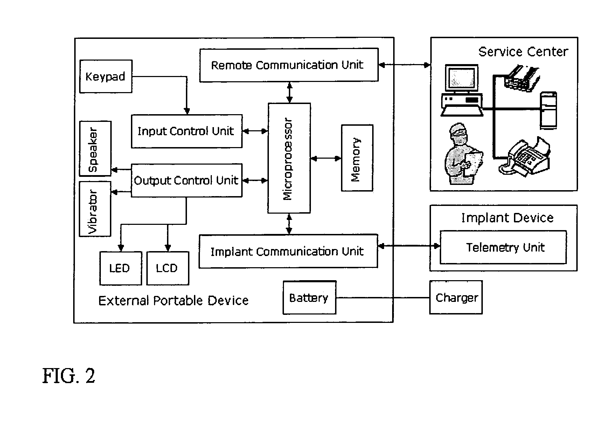

Referring now to FIG. 2, it illustrates the block diagram of the external portable device and its interfaces with the implantable atrial defibrillator and the remote service centre.

The overall functioning of the external portable device is controlled by its microprocessor, which reads and performs instructions stored in its associated memory. The instructions stored in memory preferably include instructions defining a communication protocol compatible with the implantable atrial defibrillator, and instructions defining a communication protocol compatible with the remote service centre.

The microprocessor of the external portal device communicates with the input unit to read from the keypad (or press switches) the patient inputted commands. One subset of the commands is designed to configure the external portable device, for example, to turn on or off certain outputs as described hereinafter, or select specific communication protocols. Another subset of the commands is designed to establish communication between the external portable device and the remote service centre through remote communication unit. For example, patient's input can command the external portable device to transmit diagnostic information (such as IEGM data) to the remote service centre, and wait to receive acknowledgement. The third subset of the commands is designed to establish communication between the external portable device and the implantable atrial defibrillator through implant communication unit (first external bidirectional data transceiver). For example, patient's input can command the external portable device to transmit corresponding commands to the implantable atrial defibrillator to initiate atrial cardioversion therapy, to disable or enable automatic atrial cardioversion therapy, to delay scheduled atrial cardioversion therapy by certain period of time (or cancel therapy by delaying therapy indefinitely), to retrieve IEGM and other diagnostic information from the implantable atrial defibrillator, and so on. The implant communication unit (first external bidirectional data transceiver) also receives the acknowledgement and related diagnostic information sent from the implantable atrial defibrillator, and conveys these data to the microprocessor for storage in the memory.

Upon receiving the warning trigger signal of AF detection from the implantable atrial defibrillator, the microprocessor of the external portable device communicates with the output unit to generate output that is perceptible by the patient. Such output can be in the form of visible message, such as the light-up or blinking of a light emitting diode (LED) or the text message displayed in a liquid crystal display (LCD), or in the form of audible message such as beep, ringing tone, or pre-recorded voice messages played by a speaker, or in the form of discernible vibration by a vibrator. According to the patient's preference, one or multiple types of warning message can be respectively turned on or off via the keypad. For example, the visible warning message can be turned on while the audible warning message can be turned off during the night if the patient chooses not to be disturbed during sleep even if the implantable atrial defibrillator detects the AF. Besides generating warning messages, some diagnostic information that is received from the implantable atrial defibrillator and stored in memory (such as the duration of the AF since its onset, the mean cycle length of the AF, and the time interval to the scheduled automatic atrial cardioversion) can also be provided to the patient in the form of visual or audible messages. If the external portable device receives the eoAF signal from the implantable atrial defibrillator indicating AF terminates, the previously generated warning message will be cleared.

The external portable device, via its remote communication unit (second external bidirectional data transceiver), can further communicate with the remote service centre. Such long-range communication apparatus can be in the form of a mobile radio network, or a fixed-line telecommunication network, or the internet, as well known in the art. Examples of such long-range communication apparatus have been taught in U.S. Pat. 6,470,215, U.S. Pat. 6,574,509, U.S. Pat. 6,622,043, all of which patents are assigned to the assignee of the present invention and incorporated herein by reference. Besides generating warning messages to the patient, the external portable device can also transmit critical diagnostic information stored in memory to the remote service centre, preferably including the duration of the AF since its onset, the mean cycle length of the AF, the number of failed ATP attempts, and the IEGM snapshot data. The remote service centre receives the diagnostic information via compatible communication protocols, then sends acknowledgement back to the external portable device, which generates visible or audible output indicating receipt of the acknowledgement. The data received by the remote service centre is stored in central database, and is promptly presented to the patient's physician or responsible expert through proper means, such as fax or email as known in the art. By reviewing the received diagnostic information, the physician can evaluate the patient's condition and provide expert advice to the patient who wishes to contact the physician before taking any action in response to the AF warning generated by the external portable device.

Lastly, the external portable device includes a battery power source, which provides power to the electrical components of the device. The battery is chargeable by connecting to an external charger.

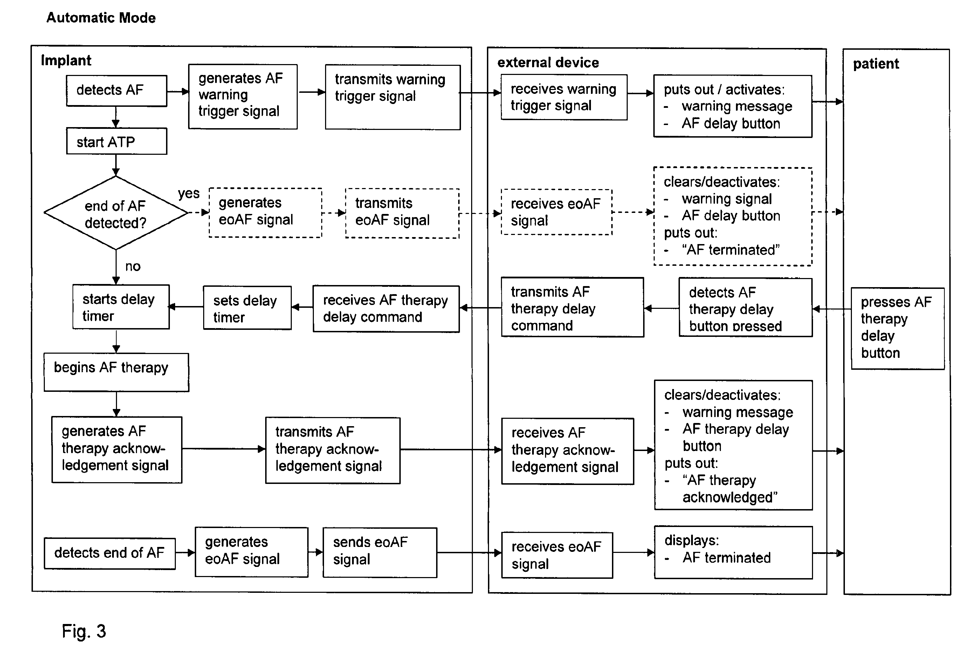

FIG. 3 illustrates the operation of the atrial defibrillation system including the implantable atrial defibrillator (implant) and the external portable device (external device) in the automatic activation mode. As already pointed out, the implant will automatically detect an atrial fibrillation by continuously monitoring the atrial rate. Upon detection of an atrial fibrillation the implant actively generates an atrial fibrillation warning trigger signal which then is transmitted to the external device. Upon reception of the warning trigger signal, the external device will put out a warning message and activate an AF delay button which enables a patient to delay or delete a scheduled atrial fibrillation therapy.

As an option, the implant is adapted to start anti-tachycardia pacing upon detection of an atrial fibrillation. Preferably, several anti-tachycardia pacing attempts are started. Should an anti-tachycardia pacing be successful and lead to an end of atrial fibrillation, the implant will generate an-end-of-atrial-fibrillation signal (eoAF signal) and transmit such signal to the external device. Upon receipt of the end of atrial fibrillation signal (eoAF signal) the external device will clear a warning signal and deactivate the atrial fibrillation delay button. Simultaneously, the external device will put out an "AF terminated" message.

As an option not further illustrated in FIG. 3, after the implant device detects an AF, it generates the warning signal only after a programmable delay, preferably also after a programmed number of ATP attempts fail to terminate the AF.

As another option not further illustrated in FIG. 3, the implant and the external device may also be adapted to retrieve and display information regarding a current atrial tachycardia or atrial fibrillation episode. In a preferred embodiment, the external device provides for the possibility of entering a data request command by the patient. Such data request command is transmitted from the external device to the implantable atrial defibrillator and causes the implantable atrial defibrillator to transmit diagnostic information to the external device the diagnostic information then is displayed by the external device to the patient.

If no end of atrial fibrillation has occurred within a scheduled period of time after detection of atrial fibrillation, the implant will trigger an atrial fibrillation therapy. This atrial fibrillation therapy may be delayed by a delay timer which is said upon receipt of an AF therapy delay command by the implant. Such AF therapy delay command would be transmitted from the external device to the implant if the patient has triggered an AF therapy delay button. Alternatively, patient may opt to cancel the schedule atrial therapy by setting an extremely long therapy delay.

AF therapy is started at the timeout of the therapy delay. If AF therapy is started, the implant generates an AF therapy acknowledgement signal and transmits such signal to the external device. Upon receipt of the AF therapy acknowledgement signal the external device will clear the AF warning message and deactivate the AF therapy delay button. Simultaneously, the external device will put out an "AF therapy acknowledge" message.

If AF therapy is successful and the implant detects an end of atrial fibrillation, the implant will generate an end of atrial fibrillation signal (eoAF signal) and send the signal to the external device. Upon receipt of said eoAF signal, the external device will display an "AF terminated" message.

In an improved embodiment not illustrated in FIG. 3, the external device may be adapted to display diagnostic and therapy information to the patient after successful termination of an atrial fibrillation episode. Such information may be displayed automatically. Alternatively, the information can be retrieved by the patient by pressing a data request command button.

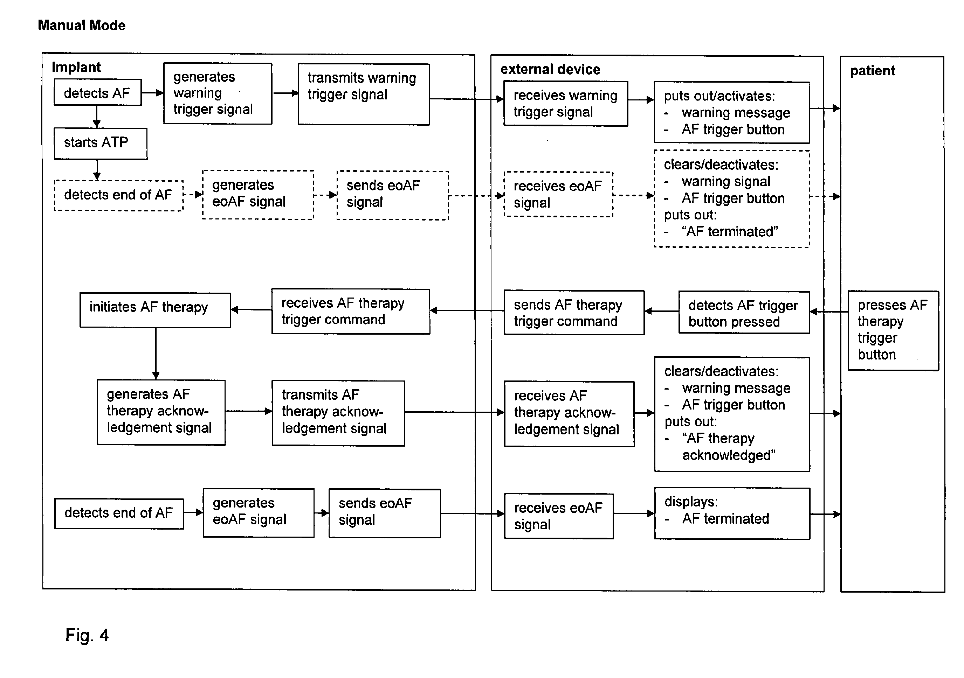

Turning out to FIG. 4 illustrating the operation of the atrial defibrillation system if the patient activation mode is selected.

Since the patient activation mode in many respect is similar to the automatic activation mode, only the differences will be discussed.

In the patient activation mode, the implant detects an atrial fibrillation the same way it would detect an atrial fibrillation in the automatic activation mode. The AF detection triggers the implant device to generate a warning signal to the patient. Alternatively, the warning signal is generated after a programmable delay, or preferably after a programmed number of ATP attempts fail to terminate the AF. Upon receiving of a warning trigger signal, the external device displays the warning message and activates an AF trigger button (instead of an AF therapy delay button). By pressing the AF trigger button, the patient can manually initiate an AF therapy. Once an AF therapy is started, the operation of the atrial defibrillation system in the patient activationmode is similar to the operation in the automatic activation mode.

Detection of an end of atrial fibrillation prior to initiating the atrial fibrillation therapy causes the external device to clear the warning signal and to deactivate the AF trigger button. In addition, the external device may put out an "AF terminated" message.

Regarding retrieval and display of diagnostic and therapy operation, the atrial defibrillation system may operate in the patient activation mode the same way as in the automatic activation mode.

Although the invention has been described above with reference to a preferred embodiment, it should be noted that such an embodiment is merely illustrative of the application of the principles of the invention.

## Claims

1. Atrial defibrillation system comprising:
- an implantable atrial defibrillator having
- an implant's control unit
- an implant's bidirectional data transceiver connected to the control unit
- an automatic atrial fibrillation detector, connected to the control unit and being adapted to receive data from and send data to an external patient device
- an atrial cardioversion/defibrillation unit connected to and being responsive to the control unit
- said implantable atrial defibrillator being adapted to operate in at least one of:
- a patient-activation mode wherein AF therapy is manually initiated by a patient via an external portable device
or
- an automatic-activation mode wherein AF therapy is automatically initiated by the implant's control unit
- an external portable device having
- a human interface including
- an output unit and
- an input unit,
- a first external bidirectional data transceiver adapted to bidirectionally communicate with the implant's bidirectional data transceiver of the implantable atrial defibrillator and being connected to the human interface
- a second external bidirectional data transceiver adapted to bidirectionally communicate with a central service centre
wherein
- the automatic atrial fibrillation detector is adapted to automatically determine the presence or absence of an atrial fibrillation and to notify the control unit accordingly
- the implant's control unit is adapted
- to trigger sending of a warning trigger signal via the implant's bidirectional data transceiver after the automatic fibrillation detector has notified the implant's control unit of an ongoing atrial fibrillation, the warning trigger signal being adapted to cause the external patient device to cause it's output unit to put out a patient discernable warning signal, and
- to trigger the atrial cardioversion/defibrillation unit
a) after a warning trigger signal was send out
and
b) if a defibrillation trigger signal is received by the implant's data transceiver after sending out of said warning trigger signal
or
if the implantable atrial defibrillator is in its automatic-activation mode and automatic triggering of the atrial cardioversion/defibrillation unit is not switched off by a patient's command
and
c) if the automatic fibrillation detector determines that the atrial fibrillation is still going on.

2. The atrial defibrillation system according to claim 1, wherein
- the implantable atrial defibrillator is adapted to send out a clearing signal if the automatic fibrillation detector determines that the atrial fibrillation has terminated after the warning trigger signal was send out,
and
- the external portable device is adapted to clear the warning signal upon receipt of the clearing signal.

3. The atrial defibrillation system according to claim 1 or 2, further including a remote service centre having means to bidirectionally communicate with the external portable device.

4. An implantable atrial defibrillator forming an implant comprising:
- a control unit,
- an implant's bidirectional data transceiver connected to the control unit,
- an automatic atrial fibrillation detector, connected to the control unit and being adapted to receive data from and to send data to an external patient device, and
- an atrial cardioversion/defibrillation unit connected to and being responsive to the control unit
wherein
- the automatic atrial fibrillation detector is adapted to automatically determine the presence or absence of an atrial fibrillation and to notify the implant's control unit accordingly
- the implant's control unit is adapted
- to trigger sending of warning trigger signal via the implant's bidirectional data transceiver after the automatic fibrillation detector has notified the implant's control unit of an ongoing atrial fibrillation, the warning trigger signal being adapted to cause the external patient device to cause the external device's output unit to put out a patient discernable warning signal, and
- to trigger the atrial cardioversion/defibrillation unit
a) after said warning trigger signal was send out
and
b) if a defibrillation trigger signal is received by the implant's data transceiver after the warning trigger signal was send out
or
if the implantable atrial defibrillator is in its automatic-activation mode and automatic triggering of the atrial cardioversion/defibrillation unit is not switched off by a patient's command
and
c) if the automatic fibrillation detector determines that the atrial fibrillation is still present.

5. The atrial defibrillator system according to claim 4, wherein
- the implantable atrial defibrillator is adapted to send out a clearing signal if the automatic fibrillation detector determines that the atrial fibrillation has terminated after a notification of an ongoing atrial fibrillation was send out.

6. The implantable atrial defibrillator according to claim 4 or 5, wherein the automatic atrial fibrillation detector is connected to an atrial sensing unit and adapted to process signals derived by the atrial sensing unit.

7. The implantable atrial defibrillator according to claim 6, wherein the atrial sensing unit is adapted to derive and record an intracardiac electrogram and the automatic atrial fibrillation detector is adapted to process said recorded intracardiac electrogram.

8. The implantable atrial defibrillator according to one of the preceding claims 4 to 7, wherein the automatic atrial fibrillation detector is adapted to determine an absence or presence of an atrial fibrillation by way of an analysis of rate, rhythm, morphology, and/or other features of the recorded atrial intracardiac electrogram.

9. The implantable atrial defibrillator according to one of the preceding claims 4 to 8, wherein the atrial cardioversion/defibrillation unit is adapted to interrogate the automatic atrial fibrillation detector and to thereby check the presence of atrial fibrillation prior to atrial cardioversion and abandon the atrial cardioversion if atrial fibrillation is not confirmed.

10. The implantable atrial defibrillator according to one of the preceding claims 4 to 9, wherein the implantable atrial defibrillator comprises a ventricular sensing unit adapted to sense ventricular signals and wherein the atrial cardioversion/defibrillation unit is adapted to deliver the cardioverting electrical energy in synchronous with a shockable R wave based on analysis of the ventricular sensed signals.

11. The implantable atrial defibrillator according to one of the preceding claims 4 to 10, wherein the implant's control unit is adapted to delay triggering of the atrial cardioversion/defibrillation unit upon receipt of a patient triggered delay command.

12. The implantable atrial defibrillator according to one of the preceding claims 4 to 11, wherein the implantable atrial defibrillator comprises an atrial stimulation unit adapted to generate atrial stimulation pulses having a strength sufficient to excitation of atrial myocardium and wherein the implant's control unit is adapted to trigger the atrial stimulation unit do deliver an anti-tachycardia pacing (ATP) after the automatic fibrillation detector has notified the implant's control unit of an ongoing atrial fibrillation.

13. The implantable atrial defibrillator according to one of the preceding claims 4 to 12, wherein the implantable atrial defibrillator comprises means to retrieve and store diagnostic information and is adapted to transmit said diagnostic information upon receipt of a data request command received via the implant's bidirectional data transceiver.

14. The implantable atrial defibrillator according to claim 13, wherein the implantable atrial defibrillator is adapted to retrieve and store device-recorded atrial intracardiac electrogram (IEGM) as diagnostic information to be transmitted to the external device upon demand.

15. An external portable device comprising
- a human interface including
- an output unit
- an input unit (command entering unit)
- a first external bidirectional data transceiver adapted to bidirectionally communicate with the implant's bidirectional data transceiver of the implantable atrial defibrillator and being connected to the human interface
- a second external bidirectional data transceiver adapted to bidirectionally communicate with a central service centre

16. The external portable device according to claim 15, wherein
the output unit is adapted to generate at least one of:
- a light-up or blinking of one or more light emitting diodes;
- a text message displayed in a liquid crystal display;
- an audible message played by a speaker; or
- a mechanical vibration generated by a vibrator.

17. The external portable device according to claim 15 or 16, wherein
the output unit is adapted to generate a warning message upon receipt of a warning trigger signal transmitted by the implantable atrial defibrillator.

18. The external portable device according to one of the preceding claims 15 to 17, wherein the input unit is adapted to allow for entering of a control command.

19. The external portable device according to claim 18, wherein the control command is one of
a therapy trigger command,
a therapy delay command
a therapy enable/disable command or
a data request command.

20. The external portable device according to claim 18 or 19, wherein the input unit is adapted activate or deactivate the possibility to enter one or more of the control commands depending upon signals received from the implantable atrial defibrillator via the first external bidirectional data transceiver, thus providing a situation adaptive human interface.
